# EUROPEAN PATENT SPECIFICATION

(11) **EP 1 015 613 B1**
(45) Date of publication and mention of the grant of the patent: **10.01.2007**
(21) Application number: 98944069.8
(22) Date of filing: 21.09.1998
(51) Int. Cl.: C12N 15/82, C07K 14/415, C12N 5/10, A01H 5/00

(54) **CONTROL POD DEHISCENCE OR SHATTER**
KONTROLLE DER SCHOTENDEHISZENZ ODER SCHOTENABWURFS
REGULATION DE LA DEHISCENCE OU DE L'ECLATEMENT DES GOUSSES

(30) Priority: 19.09.1997 GB 9720039
(43) Date of publication of application: 05.07.2000
(73) Proprietor: BIOGEMMA UK LIMITED, Cambridge CB4 4GZ (GB)
(72) Inventor: PAUL, Wyatt, Nickerson Biogemma Limited, Cambridge CB4 4GZ (GB); ROBERTS, Jeremy Alan, University of Nottingham, Loughbourough, Leicestershire LE 12 5RD (GB); WHITELAW, Catherine, BARC-West Building 006, r.208, Beltsville, MD 20705 (US)
(74) Representative: Flesselles, Bruno F.G.
(86) International application number: PCT/GB1998/002850
(87) International publication number: WO 1999/015681

(56) References cited:
- WO-A-94/23043
- WO-A-96/30529
- WO-A-97/13865
- JENKINS E S ET AL: "MOLECULAR CHARACTERISATION OF BRASSICA NAPUS POD DEHISCENCE" JOURNAL OF EXPERIMENTAL BOTANY, 1 January 1995, page 42 XP000570274

## Description

This invention relates to the control of pod dehiscence or shatter.

In the process of pod dehiscence, or shatter as it is commonly termed, degradation and separation of cell walls occurs along a discrete layer of cells, termed the dehiscence zone, and a localised increase in the activity of cellulase has been reported prior to the onset of dehiscence (Meakin and Roberts *J. Exp. Bot.* 41(229) 995-1002 (1990) and *J. Exp. Bot.* 41(229) 1003-1011 (1990)). This process is agronomically important because it may result in the premature shedding of seed before the crop can be harvested. Adverse weather conditions can exacerbate the process resulting in a greater than 50% loss of seed. This loss of seed not only has a dramatic effect on yield but also results in the emergence of the crop as a weed in the subsequent growing season.

Attempts to solve this problem over the last 20 years have focused on the breeding of shatter-resistant varieties. The most commonly used method is by trying to introduce germplasm from related species by interspecific hybridisation. Related species such as *B. nigra, B. juncea* and *B. campestris* have been used for this purpose but resulting plants from these crosses are frequently sterile and lose favourable characteristics which have to be regained by back crossing. This is both time consuming and laborious. The interspecific hybridisation strategy also has to cope with transferring two or more genes which are recessive in action into each of the breeding lines. Indeed, even within *B. campestris,* different genetic backgrounds have revealed different numbers of genes to be important in shatter resistance. This has necessitated breeders performing test crosses at each generation during the attempt to produce elite material. These difficulties have been compounded by the fact that shattering is a difficult and time-consuming trait to assess in the field. All these factors may account for the fact that the conventional breeding approach has made no progress over the last twenty years.

Other methods employed to try and alleviate the problem include chemicals, in the form of desiccants and pod sealants. The most widely used method to try and prevent seed loss is the mechanical technique of swathing in order to get uniform desiccation of the crop and reduce shattering by wind which occurs in the upright crop.

WO 96/30529 discloses the use of nucleic acid sequences coding for polygalacturonase in the control of dehiscence in plants. Journal of Experimental Botany 1995, p42, XP000570274(P6.72) discloses the molecular characterisation of *Brassica napus* pod dehiscence.

The present invention provides, by the use of recombinant technology, a further advantageous means for the control of pod dehiscence.

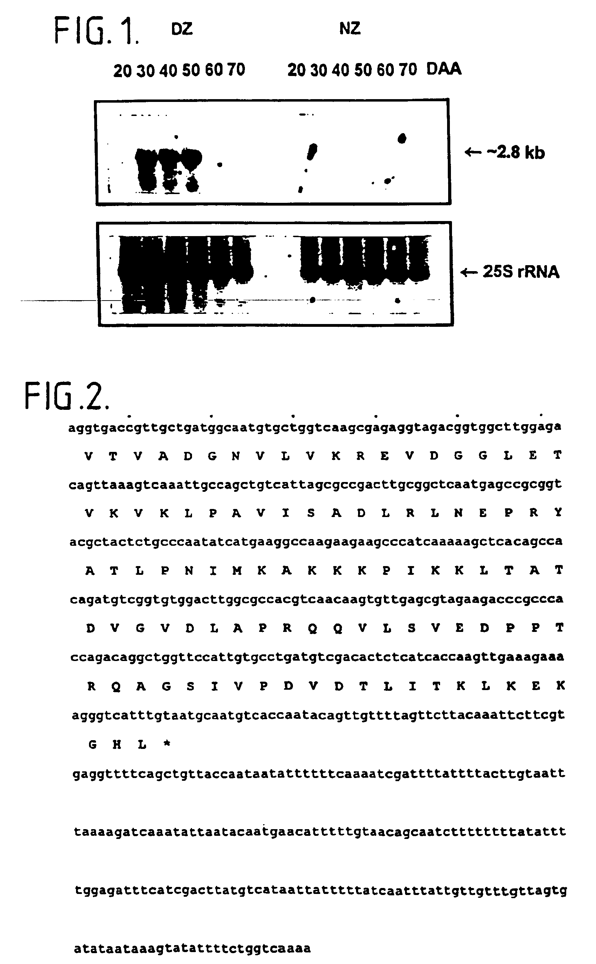

According to a first aspect of the present invention, there is provided nucleic acid, preferably recombinant or isolated nucleic acid sequence comprising the sequence as shown in figure 2 or a sequence substantially homologous thereto. The nucleic acid is and/or the polypeptide which it encodes is involved in the process of pod dehiscence.

The invention has application to all crops that lose seed pre-harvest because of cell separation events. An economically important crop to which the invention applies is *Brassica napus.*

Recombinant or isolated nucleic acid sequences which are within the scope of the invention include those which, by virtue of the degeneracy of the genetic code, also code for the amino acid sequence indicated in figure 2. Nucleic acid sequences which are substantially homologous to nucleic acid sequences encoding the amino acid sequence shown in figure 2 also constitute preferred embodiments of the invention. "Substantial homology" may be assessed either at the nucleic acid level or at the amino acid level.

Any substantially homologous sequence should be at least 95% identical to the coding sequence in Figure 2 at the nucleic acid residue level, using the default parameters of the BLAST 2.0 program of the National Centre for Biotechnology Information (available at http:www.ncbi.nlm.nih.gov or Altschul, S. F., *et al.,* Nucleic Acids Research, September 1997, 25(17), 3389-3402). Also covered by the present invention are sequences which comprise regions of complementary sequences to any sequence described above (that is any complementary sequence to the sequence shown in Figure 2, or a substantial homologue of such a sequence (as defined above)). Any fragment of the sequence (or of a sequence substantially homologous thereto, or of a complementary sequence thereto, including homologues) is at least 20 bases, preferably at least 30, 40, 50 or 60 bases in length.

At the nucleic acid level, sequences having substantial homology may also be regarded as those which are complementary to any of the previously described sequences (ie a sequence comprising a sequence as shown in Figure 2, or a subsantial homologue thereof, or a fragment thereof). The invention can be used to identify sequences which hybridise to the nucleic acid sequences shown in Figure 2 under stringent conditions (for example at 35 to 65°C in a salt solution of about 0.9M) or conditions described in Plant Genetic Transformation and Gene Expression: A Laboratory Manual, Ed. Draper, J., *et al.,* 1988, Blackwell Scientific Publications, pp 252-255, modified as follows: prehybridization, hybridization and washes at 55 to 65°C, final washes (with 0.5X SSC, 0.1% SDS) omitted.

All references in this text to sequences includes within its scope substantially homologous sequences.

Fragments of the isolated or recombinant nucleic acid according to the invention are those which are functional and/or those which can be used in the regulation of plant dehiscence and/or those which can be used as tools, such as probes to identify homologous sequences (including orthologous sequences). Preferably, fragments are at least 20 bases, preferably at least 30, 40, 50, 60, 70, 80, or 90 bases in length. The invention most preferably relates to the full-length dehiscence zone protein a part of which is herein designated DZ15 as well as a nucleic acid sequence coding therefore, optionally including the promoter sequence.

Sometimes, the nucleic acid of the invention could include a promoter or other regulatory sequence which naturally controls its expression. Since the sequence is expressed during dehiscence, such promoters are themselves useful in controlling this process.

While promoters as described above may drive DNA encoding an enzyme, they may alternatively drive DNA whose transcription product is itself deleterious. Examples of such transcription products include antisense RNA and ribozymes.

As far as antisense nucleic acid is concerned, introducing the coding region of a gene in the reverse orientation to that found in nature can result in the downregulation of the gene and hence the production of less or none of the gene product. The RNA transcribed from antisense DNA is capable of binding to, and destroying the function of, a sense RNA of the sequence normally found in the cell, thereby disrupting function. Examples of such antisense DNAs are the antisense DNAs of the sequence shown in Figure 2 and the sequence as claimed in the invention. Since this gene is normally expressed in the dehiscence zone, antisense to it may be expected to disrupt normal dehiscence. Downregulation of the protein according to the present invention can be achieved by downregulation of the gene by partial or full (transwitch) sense expression.

Ribozymes are RNA "enzymes" capable of highly specific cleavage against a given target sequence (Haseloff and Gerlach, *Nature* 334 585-591 (1988)). The present invention also relates to ribozymes which are involved in or disrupt the correct transcription and expression of the dehiscence protein of the present invention.

Promoters useful as described above may be located in genomic libraries using, for example, probe sequences taken from the nucleic acid sequence of Figure 2 or as defined in the invention (by standard procedures as described below).

In another aspect, nucleic acid useful in the invention includes that which, when introduced into a plant, prevents or otherwise interferes with normal dehiscence by interfering with the normal plant expression. Of course, dehiscence-specific promoters, as discussed above, may be useful in this aspect of the invention.

However, there is a broader dimension which must be considered: antisense DNA or ribozyme-encoding DNA need not be driven by dehiscence-specific promoters. Instead, they could be driven by constitutive or other promoters (such as for example the nos, rubisco or plastocyanin promoter). If the sense gene is only expressed in the pod, there will, with an antisense approach, be no pleiotropic effects on plant development, and only the development of the dehiscence zone will be disrupted.

Antisense technology and ribozyme technologies have already found application in other areas of plant molecular biology. For example, antisense technology has been used to control tomato fruit ripening. Ribozyme technology has been used to control viral infection of melons. While DNA or RNA in accordance with this feature of the invention generally interferes with the plant's proper expression, in preferred embodiments expression is substantially prevented.

Another important aspect of the present invention is ability to work with those nucleic acids which hybridise under stringent conditions to the nucleic acid sequences of the invention. For example, nucleic acid fragments are useful for probing for similar genes involved in dehiscence. For example, an *Arabidopsis* or other gene library may be probed. Fragments of the nucleic acid sequence shown in Figure 2, of at least 10, 20, 30, 40 or 50 more nucleotides may be used. Many useful probes are from 15 to 20 nucleotides in length.

In preferred embodiments of DNA sequences of this invention, 3'-transcription regulation signals, including a polyadenylation signal, may be provided. Preferred 3'-transcription regulation signals may be derived from the cauliflower mosaic virus 35S gene. It should be recognised that other 3'-transcription regulation signals could also be used.

The nucleic acid of the invention, preferably recombinant DNA in accordance with the invention may be in the form of a vector. The vector may for example be a plasmid, cosmid or phage. Vectors will frequently include one or more selectable markers to enable selection of cells transfected (or transformed: the terms are used interchangeably in this specification) with them and, preferably, to enable selection of cells harbouring vectors incorporating heterologous DNA. Appropriate start and stop signals will generally be present. Additionally, if the vector is intended for expression, sufficient regulatory sequences to drive expression will be present; however, DNA in accordance with the invention will generally be expressed in plant cells, and so microbial host expression would not be among the primary objectives of the invention, although it is not ruled out. Vectors not including regulatory sequences are useful as cloning vectors.

Cloning vectors can be introduced into *E. coli* or another suitable host which facilitate their manipulation. According to another aspect of the invention, there is therefore provided a host cell transfected or transformed with DNA as described above.

DNA in accordance with the invention can be prepared by any convenient method involving coupling together successive nucleotides, and/or ligating oligo- and/or poly-nucleotides, including *in vitro* processes, but recombinant DNA technology forms the method of choice.

In the embodiments of the invention relating to dehiscence, the invention has application to all crops that lose seed pre-harvest because of cell separation events. An economically important crop to which the invention applies is *Brassica napus.* The invention is also particularly relevant to plants that develop dry fruits, including Brassica, Synapis and other genera of the Brassicaceae, soybean and other Leguminous species, Cuphea and sesame.

In preferred embodiments of DNA sequences of this invention, 3'-transcription regulation signals, including a polyadenylation signal, may be provided. Preferred 3'-transcription regulation signals may be derived from the cauliflower mosaic virus 35S gene. It should be recognised that other 3'-transcription regulation signals could also be used. Derivation of the full length sequence of DZ15 as well as 3' and 5' sequences can be carried out by standard procedures well known in the art. As a general procedure, sequences 5' to the coding sequence can be identified using the following strategy: 1) use the DZ15 sequence (or part thereof) to probe a genomic library to obtain the full length clone, 2) locate the first ATG of the full length clone, 3) [optional] compare with other known sequences (such as on databases) to confirm the position of the ATG, 4) design primers to PCR a fragment from the ATG to a region upstream, preferably 1kb or more. Sequences 3' can be identified by an analogous process.

Ultimately, DNA in accordance with the invention will where appropriate be introduced into plant cells, by any suitable means. According to a further aspect of the invention there is provided a plant cell including DNA in accordance with the first aspect of the invention as described above.

Preferably, DNA is transformed into plant cells using a disarmed Ti-plasmid vector and carried by *Agrobacterium* by procedures known in the art, for example as described in EP-A-0116718 and EP-A-0270822. Alternatively, the foreign DNA could be introduced directly into plant cells using an electrical discharge apparatus. This method is preferred where *Agrobacterium* is ineffective, for example where the recipient plant is monocotyledonous. Any other method that provides for the stable incorporation of the DNA within the nuclear DNA of any plant cell of any species would also be suitable. This includes species of plant which are not currently capable of genetic transformation.

Preferably DNA in accordance with the invention also contains a second chimeric gene (a "marker" gene) that enables a transformed plant containing the foreign DNA to be easily distinguished from other plants that do not contain the foreign DNA. Examples of such a marker gene include antibiotic resistance (Herrera-Estrella *et al. , EMBO J.* **2**(6) 987-95 (1983) and Herrera-Estrella *et al., Nature* **303** 209-13 (1983)), herbicide resistance (EP-A-0242246) and glucuronidase (GUS) expression (EP-A-0344029). Expression of the marker gene is preferably controlled by a second promoter which allows expression in cells other than the dehiscence zone, thus allowing selection of cells or tissue containing the marker at any stage of regeneration of the plant. The preferred second promoter is derived from the gene which encodes the 35S subunit of Cauliflower Mosaic Virus (CaMV) coat protein. However any other suitable second promoter could be used.

A whole plant can be regenerated from a single transformed plant cell, and the invention therefore provides transgenic plants (or parts of them, such as propagating material) including DNA in accordance with the invention as described above. The regeneration can proceed by known methods.

The invention leads to a newly isolated protein/polypeptide which is preferentially or exclusively expressed in dehiscence zones. Such a protein is that whose amino acid sequence is encoded, at least in part, by the sequence as set out in claim 1. This aspect of using the invention provides a recombinant or isolated protein or polypeptide comprising the amino acid sequence as set out in Figure 2, or a sequence substantially homologous thereto, or a fragment of the amino acid sequence set out in Figure 2. Any substantially homologous sequence should be at least above 60% identical, preferably through 70%, 75%, 80%, 85%, 87%, 90%, 95% identical or at least above 76% similar, preferably through 80%, 85%, 90%, 95% similar (all) at the amino acid residue level, using the default parameters of the BLAST 2.0 program of the National Centre for Biotechnology Information (available at http:www.ncbi.nlm.nih.gov and also at the reference cited above alongside the web-site details). Any fragment should be of 6, preferably up to 10, 15 or 20 amino acid residues in length, of such a sequence. The invention also leads to a protein sequence which comprises the amino acid sequence as set out in Figure 2 and which is substantially the complete protein.

The polypeptide /protein sequences are particularly useful in developing tools, such as antibodies having specificity for identical or substantially homologous proteins/polypeptide sequences (including orthologous sequences) for further isolation or for identification of relevant sequences.

The present invention also provides a nucleic acid sequence which encodes the amino acid sequence set out in Figure 2. The nucleic acid sequence may be the sequence set out in Figure 2 or may differ, taking into account the degenerate nucleic acid code.

In yet a further aspect the invention provides the use of a recombinant or isolated nucleic acid sequence as described according to the first aspect, in the control of pod dehiscence. The use, in particular, is for the transformation of a plant, or of a plant part (such as propagating material) or a cell, to produce a transformed plant, plant part or plant cell, optionally to product plant propagating material.

The invention also provides a method of regulating dehiscence, which method comprises transforming propagating material from a plant with a nucleic acid sequence according to the first aspect of the invention.

Many of the techniques referred to herein use, or can use, hybridization to identify homologous sequences, or to probe for complementary sequences. Standard hybridization techniques can be used. For example, promoter sequences of interest can be used to identify and isolate substantially homologous promoters from other plants. Typically, a sequence is used, possibly a fragment thereof from 15 to 45 base pairs to hybridize to sequences from a suitable library under stringent conditions. Suitable conditions may be those described in Plant Genetic Transformation and Gene Expression: A Laboratory Manual, Ed. Draper, J., *et al.,* 1988, Blackwell Scientific Publications, pp 252-255, modified as follows: prehybridization, hybridization and washes at 55 to 65°C, final washes (with 0.5X SSC, 0.1% SDS) omitted.

Preferred features and details of each aspect (independent claim type) of the invention are as for each other aspect *mutatis mutandis.*

The present invention is supported and described with reference to the figures, in which:
Figure 1 shows Northern analysis of the expression of DZ15 in *B. napus* pods. A DZ15 riboprobe was generated using T3 DNA polymerase and used to probe RNA isolated from pod dehiscence zones (DZ) or from pod valves lacking DZ (NZ). The blot was reprobed with the control probe 25S rRNA, to ensure that all lanes contained RNA.
Figure 2 shows the DNA sequence and putative amino acid sequence of DZ15.

The invention will now be illustrated by the following Examples.

### EXAMPLE 1 - Identification and cloning of DZ15

### Plant Material

Seeds of *B. napus* cv Rafal were grown as described by Meakin and Roberts, (*J. Exp. Bot.* 41(229) 995-1002 (1990)) with the following modifications. Single seedlings were potted into 10 cm pots containing Levington M2 compost, and after vernalisation for 6 weeks, were re-potted into 21 cm pots. Infection by powdery mildew or aphids was controlled by the application of Safers fungicide and insecticide. At anthesis, tags were applied daily to record flower opening. This procedure facilitated accurate age determination of each pod. Pods were harvested at various days after anthesis (DAA). The dehiscence zone was excised from the non-zone material and seed using a scalpel blade using the method of Meakin and Roberts (*J. Exp. Bot.* 41: 1003-1011 (1990)) and immediately frozen in liquid N₂ prior to storage at -70°C.

### RNA Isolation

All chemicals were molecular biology grade and bought from either Sigma Chemical Ltd (Dorset, UK), or ICN Biomedicals. Total RNA was extracted using the polysomal extraction method of Christoffersen and Laties, *Proc. Natl. Acad. Sci.* 79 4060-4063 (1982), with the following alterations. The plant material was ground to a powder in liquid N₂ and then in 10 volumes of extraction buffer (200 mM Tris-acetate [pH 8.2], 100 mM magnesium acetate, 20 mM potassium acetate, 20 mM EDTA, 5% w/v sucrose, after sterilisation 2-mercaptoethanol was added to 15 mM and cycloheximide added to a final concentration of 0.1 mg ml⁻¹). The supernatant was then layered over 8 ml 1M sucrose made with extraction buffer and centrifuged in a KONTRON^{™} (Switzerland) TFT 70.38 rotor at 45,000 rpm (150,000 g) for 2 hr at 2°C in a Kontron CENTRIKON^{™} T-1065 ultra-centrifuge. Pellets were then resuspended in 500 µl 0.1M sodium acetate, 0.1 % SDS, pH 6.0 and phenol/chloroform (1:1 v/v) extracted and the total RNA precipitated. mRNA was isolated from the total population of nucleic acids extracted from the dehiscence zone and non-zone tissue at 30 to 50 DAA pods, using the Poly(A) quick mRNA purification kit (Stratagene, cambridge UK), and was used to make 1st strand cDNA using reverse transcriptase.

### Differential display

This was performed essentially as described by Liang and Pardee (1992) [Liang, P. and Pardee, A. B. (1992) Differential display of eukaryotic messenger RNA by means of the polymerase chain reaction. *Science* 257, 967-971] using RNA extracted from 50 DAA (days after anthesis) pod dehiscence zones and non-zones. First strand cDNA copies of the RNAs (50 DAA DZ (non-dehiscence zone)/NZ (dehiscence zone) were made using 50U M-MLV (Moloney Murine Leukemia Virus) reverse transcriptase (50U/*µ*L) (Stratagene) in a 20*µ*L reaction containing 1x M-MLV buffer, 2.5mM dNTPs (Pharmacia), 1*µ*g RNA, 30U RNAse inhibitor (Promega) and 10*µ*M oligo dT anchor primer 8 (5'-TTTTTTTTTTTTGA-3'). The reaction conditions were as follows: 65°C for 5 minutes, 37°C for 90 minutes and 95°C for 5 minutes. Following first strand cDNA synthesis, 60*µ*L dH2O were added and the samples were either used directly for PCR or stored at -20°C.

For PCR, 2µL cDNA were used as template in a 20µL reaction containing 1x PCR buffer, 1mM MgCl2, 2µM dNTPs, 10µM oligo dT anchor primer 8 (5'-TTTTTTTTTTTTGA-3'), 2.5µM arbitrary primer C (5'-AGGTGACCGT-3'), 0.5µL 35S-dATP (> 1000 Ci/mmol) (Amersham) and 1U *Taq* DNA polymerase (5U/µL) (Gibco BRL). The thermocycling conditions were as follows: 40 cycles of 94°C for 30 seconds, 40°C for 2 minutes, 720C for 30 seconds followed by 72°C for 5 minutes. The PCR products were fractionated on a 5 % polyacrylamide/7M urea gel after addition of 5µL loading buffer (95% (v/v) formamide, 20mM EDTA, 0.05% (w/v) xylene cyanol, 0.05% (w/v) bromophenol blue) to each sample. Following electrophoresis the gel was dried at 80°C under vacuum for 1 hour then exposed to X-ray film (BioMax-MR, Kodak) in a light tight cassette for 48 hours. The dried gel and autoradiogram were aligned so that bands that appeared in the DZ and not in NZ could be cut out and the DNA eluted according to Liang et al. (1995) [Liang, P., Bauer, D., Averboukh, L., Warthoe, P., Rohrwild, M., Muller, H., Strauss, M. and Pardee, A. B. (1995) Analysis of altered gene expression by differential display. *Methods in Enzymology* 254, 304-321.]. The eluted PCR products (4*µ*L) were reamplified in a 40*µ*L reaction containing 1x PCR buffer, 1mM MgCl2, 20*µ*M dNTPs, 10µM oligo dT anchor primer 8 (5'-TTTTTTTTTTTTGA-3'), 2.5µM arbitrary primer C (5'-AGGTGACCGT-3') and 2U *Taq* DNA polymerase (5U/*µ*L) (Gibco BRL) using the following thermocycling conditions: 40 cycles of 94°C for 30 seconds, 40°C for 2 minutes, 72°C for 30 seconds followed by 72°C for 5 minutes. The resulting PCR product was cloned into the TA cloning vector (Invitrogen) and sequenced [Figure 1]. In order to prepare an antisense strand-specific riboprobe, the PCR product was subcloned into pBluescript (Stratagene).

### Expression pattern and analysis of DZ15.

Northern analysis using an antisense strand-specific riboprobe to the DZ15 PCR product, showed that DZ15 hybridised to a transcript of size 2.8kb to 3.4kb which was expressed in the DZ of 30-50 DAA pods (Figure 1). Minimal expression was observed in the pod NZ (Figure 1). A full-length DZ15 cDNA can be obtained by further screening of a cDNA library or using 5' RACE. Similarly the DZ15 gene and promoter can be obtained by standard techniques by screening a genomic library; for example a genomic library from *B.napus* or from a relative such as *Arabidopsis thaliana* can be employed.

The sequenced DZ15 PCR product (Figure 2) exhibits no extensive homology with any sequences in DNA databases.

### EXAMPLE 2

### Production of shatter-resistant B.napus plants by antisense downregulation of DZ15

Any promoter that is expressed in the pod DZ during the time interval that DZ15 is expressed would be useful in a strategy to downregulate expression of the DZ15 gene product. Such a promoter is the DZ15 promoter. The DZ15 promoter was therefore obtained from an *A.thaliana* genomic library and linked to the DZ15 PCR product in a manner such that the DZ15 PCR fragment was in the antisense orientation. The resultant pDZ15-antiDZ15 gene was cloned into the binary vector pSCV nos nptII (SCV nos nptII is a derivative of pSCV1 (Firek *et al.* (1993), Plant Molecular Biology 22,129-142) which contains a nos promoter driving a Kanamycin resistence gene, cloned between the EcoRV and EcoRI sites of pSCV1). This binary construct was transformed into *B.napus* (var. Westar) by agrobacterial transformation essentially as described in Moloney M *et al.,* (1989) Plant Cell Reports 8, 238-242. A proportion of the resulting *B.napus* plants were found not to express DZ15 and consequently be resistant to pod shatter.

## Claims

1. A recombinant or isolated nucleic acid comprising the sequence as shown in figure 2, or a sequence at least 95 % homologous thereto or encoding the protein shown in figure 2 wherein said nucleic acid can be used to control shatter or pod dehiscence in *Brassica napus* plants.

2. A nucleic acid as claimed in claim 1, which includes a promoter or other regulatory sequence which controls expression of the sequence.

3. A recombinant or isolated nucleic acid which is antisense to a nucleic acid as defined in claim 1.

4. A nucleic acid as claimed in any one of claims 1 to 3 which is in the form of a vector.

5. A nucleic acid as defined in any one of claims 1 to 4 which also includes a second chimeric or marker gene.

6. A nucleic acid as claimed in claim 5, wherein the second gene is under the control of a second promoter which allows expression at any stage of plant regeneration.

7. A nucleic acid as claimed in claim 6, wherein the second promoter is the nos promoter.

8. A host cell transformed or transfected with nucleic acid as claimed in any one of claims 1 to 7.

9. A plant cell which includes the nucleic acid as claimed in any one of claims 1 to 7.

10. A whole part or part of a plant, comprising a cell as claimed in claim 9.

11. A transgenic plant which includes a nucleic acid as defined in any one of claims 1 to 7.

12. Propagating material derived from a plant as claimed in claim 11, wherein said material includes nucleic acid as defined in any one of claims 1 to 7.

13. A method of regulating dehiscence, which comprises the step of transforming or transfecting propagating material from a plant with a nucleic acid as defined in any one of claims 1 to 7.

14. A method as claimed in claim 13 for reducing or preventing dehiscence, wherein the nucleic acid is as defined in claim 3.

15. The use of a recombinant or isolated nucleic acid as claimed in any one of claims 1 to 7 in the control of dehiscence.

16. The use as claimed in claim 15 wherein the nucleic acid is used to transform a plant or plant propagating material to produce a transformed plant or plant propagating material.

## Patentansprüche

1. Rekombinante oder isolierte Nukleinsäure mit der Sequenz gemäß Abbildung 2 oder einer Sequenz, die zu mindestens 95% zu dieser Sequenz homolog ist, oder die das Protein gemäß Abbildung 2 codiert, wobei die Nukleinsäure für die Kontrolle des Samenausfalls bzw. der Schotendehiszenz bei *Brassica napus*-Pflanzen verwendet werden kann.

2. Nukleinsäure nach Anspruch 1, die einen Promoter oder eine sonstige Regulationssequenz, der/die die Expression der Sequenz kontrolliert, beinhaltet.

3. Rekombinante oder isolierte Nukleinsäure, die zu einer Nukleinsäure nach Anspruch 1 antisense ist.

4. Nukleinsäure nach einem der Ansprüche 1 bis 3, die in Vektorform vorliegt.

5. Nukleinsäure nach einem der Ansprüche 1 bis 4, die außerdem ein zweites chimäres Gen oder Markergen beinhaltet.

6. Nukleinsäure nach Anspruch 5, wobei das zweite Gen unter der Kontrolle eines zweiten Promoters steht, der die Expression zu einem beliebigen Stadium der Pflanzenregeneration gestattet.

7. Nukleinsäure nach Anspruch 6, wobei es sich bei dem zweiten Promoter um den nos-Promoter handelt.

8. Wirtszelle, die mit der Nukleinsäure nach einem der Ansprüche 1 bis 7 transformiert oder transfiziert ist.

9. Pflanzenzelle, die die Nukleinsäure nach einem der Ansprüche 1 bis 7 beinhaltet.

10. Ganze Pflanze oder Teil einer Pflanze, die/der eine Zelle nach Anspruch 9 enthält.

11. Transgene Pflanze, die eine Nukleinsäure nach einem der Ansprüche 1 bis 7 enthält.

12. Vermehrungsmaterial, das von einer Pflanze nach Anspruch 11 stammt, wobei das Material eine Nukleinsäure nach einem der Ansprüche 1 bis 7 enthält.

13. Verfahren zur Regulation der Dehiszenz, das den Schritt umfaßt, daß man Vermehrungsmaterial von einer Pflanze mit einer Nukleinsäure nach einem der Ansprüche 1 bis 7 transformiert oder transfiziert.

14. Verfahren nach Anspruch 13 zur Verringerung bzw. zum Verhindern von Dehiszenz, wobei die Nukleinsäure gemäß Anspruch 3 ist.

15. Verwendung einer rekombinanten oder isolierten Nukleinsäure nach einem der Ansprüche 1 bis 7 für die Dehiszenzkontrolle.

16. Verwendung nach Anspruch 15, wobei die Nukleinsäure für die Transformation einer Pflanze oder eines Pflanzenvermehrungsmaterials verwendet wird, um zu einer transformierten Pflanze bzw. einem transformierten Pflanzenvermehrungsmaterial zu gelangen.

## Revendications

1. Acide nucléique recombinant ou isolé comprenant la séquence telle que présentée en figure 2, ou une séquence homologue à au moins 95 % à celle-ci ou codant pour la protéine présentée en figure 2, **caractérisé en ce que** ledit acide nucléique peut être utilisé pour contrôler l'éclatement ou la déhiscence des gousses chez des plantes de *Brassica napus.*

2. Acide nucléique selon la revendication 1, qui inclut un promoteur ou une autre séquence régulatrice qui contrôle l'expression de la séquence.

3. Acide nucléique recombinant ou isolé qui est antisens par rapport à un acide nucléique tel que défini dans la revendication 1.

4. Acide nucléique selon l'une quelconque des revendications 1 à 3, qui est sous la forme d'un vecteur.

5. Acide nucléique selon l'une quelconque des revendications 1 à 4, qui inclut également un deuxième gène chimère ou marqueur.

6. Acide nucléique selon la revendication 5, **caractérisé en ce que** le deuxième gène est sous le contrôle d'un deuxième promoteur qui permet l'expression à une étape quelconque de la régénération d'une plante.

7. Acide nucléique selon la revendication 6, **caractérisé en ce que** le deuxième promoteur est le promoteur nos.

8. Cellule hôte transformée ou transfectée par un acide nucléique selon l'une quelconque des revendications 1 à 7.

9. Cellule végétale qui inclut l'acide nucléique selon l'une quelconque des revendications 1 à 7.

10. Plante entière ou partie d'une plante comprenant une cellule selon la revendication 9.

11. Plante transgénique qui inclut un acide nucléique tel que défini dans l'une quelconque des revendications 1 à 7.

12. Matériel de propagation dérivé d'une plante selon la revendication 11, **caractérisé en ce que** ledit matériel inclut un acide nucléique tel que défini dans l'une quelconque des revendications 1 à 7.

13. Procédé de régulation de la déhiscence, qui comprend l'étape de transformation ou de transfection d'un matériel de propagation issu d'une plante par un acide nucléique tel que défini dans l'une quelconque des revendications 1 à 7.

14. Procédé selon la revendication 13 destiné à réduire ou à empêcher la déhiscence, **caractérisé en ce que** l'acide nucléique est tel que défini dans la revendication 3.

15. Utilisation d'un acide nucléique recombinant ou isolé selon l'une quelconque des revendications 1 à 7, dans le contrôle de la déhiscence.

16. Utilisation selon la revendication 15, **caractérisée en ce que** l'acide nucléique est utilisé pour transformer une plante ou un matériel de propagation végétal, afin de produire une plante ou un matériel de propagation végétal transformés.


### SEQUENCE LISTING

(1) GENERAL INFORMATION:
   (i) APPLICANT:
      (A) NAME: Nickerson Biocem Limited
      (B) STREET: Cambridge science park, Milton Road
      (C) CITY: Cambridge
      (E) COUNTRY: Great Britain
      (F) POSTAL CODE (ZIP): CB4 4GZ

      (A) NAME: Wyatt Paul, c/o Nickerson Biocem Limited
      (B) STREET: Cambridge Science Park, Milton Road
      (C) CITY: Cambridge
      (E) COUNTRY: Great Britain
      (F) POSTAL CODE (ZIP): CB4 4GZ

      (A) NAME: Jeremy Alan Roberts, c/o University of Nottingham, Division of Plant Science
      (B) STREET: Sutton Bonington Campus
      (C) CITY: Loughbourough
      (D) STATE: Leicestershire
      (E) COUNTRY: Great Britain
      (F) POSTAL CODE (ZIP): LE12 5RD

      (A) NAME: Catherine Whitelaw, c/o University of Nottingham, Division of Plant Science
      (B) STREET: Sutton Bonington Campus
      (C) CITY: Loughbourough
      (D) STATE: Leicestershire
      (E) COUNTRY: Great Britain
      (F) POSTAL CODE (ZIP): LE12 5RD
   (ii) TITLE OF INVENTION: Control
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: WO PCT/GB98/02850
(2) INFORMATION FOR SEQ ID NO: 1:
   (i) SEQUENCE CHARACTERISTICS:
      (A) LENGTH: 569 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 1:
   AGGTGACCGT TGCTGATGGC AATGTGCTGG TCAAGCGAGA GGTAGACGGT GGCTTGGAGA      60
   CAGTTAAAGT CAAATTGCCA GCTGTCATTA GCGCCGACTT GCGGCTCAAT GAGCCGCGGT     120
   ACGCTACTCT GCCCAATATC ATGAAGGCCA AGAAGAAGCC CATCAAAAAG CTCACAGCCA     180
   CAGATGTCGG TGTGGACTTG GCGCCACGTC AACAAGTGTT GAGCGTAGAA GACCCGCCCA     240
   CCAGACAGGC TGGTTCCATT GTGCCTGATG TCGACACTCT CATCACCAAG TTGAAAGAAA     300
   AGGGTCATTT GTAATGCAAT GTCACCAATA CAGTTGTTTT AGTTCTTACA AATTCTTCGT     360
   GAGGTTTTCA GCTGTTACCA ATAATATTTT TTCAAAATCG ATTTTATTTT ACTTGTAATT     420
   TAAAAGATCA AATATTAATA CAATGAACAT TTTTGTAACA GCAATCTTTT TTTTATATTT     480
   TGGAGATTTC ATCGACTTAT GTCATAATTA TTTTTATCAA TTTATTGTTG TTTGTTAGTG     540
   ATATAATAAA GTATATTTTC TGGTCAAAA                                       569
(2) INFORMATION FOR SEQ ID NO: 2:
   (i) SEQUENCE CHARACTERISTICS:
      (A) LENGTH: 103 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 2:
   Val Thr Val Ala Asp Gly Asn Val Leu Val Lys Arg Glu Val Asp Gly
   1               5                   10                  15
   Gly Leu Glu Thr Val Lys Val Lys Leu Pro Ala Val Ile Ser Ala Asp
               20                  25                  30
   Leu Arg Leu Asn Glu Pro Arg Tyr Ala Thr Leu Pro Asn Ile Met Lys
           35                  40                  45
   Ala Lys Lys Lys Pro Ile Lys Lys Leu Thr Ala Thr Asp Val Gly Val
       50                  55                  60
   Asp Leu Ala Pro Arg Gln Gln Val Leu Ser Val Glu Asp Pro Pro Thr
   65                  70                  75                  80
   Arg Gln Ala Gly Ser Ile Val Pro Asp Val Asp Thr Leu Ile Thr Lys
                   85                  90                  95
   Leu Lys Glu Lys Gly His Leu
               100
(2) INFORMATION FOR SEQ ID NO: 3:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 3:
      TTTTTTTTTT TTGA 14
(2) INFORMATION FOR SEQ ID NO: 4:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 4:
      AGGTGACCGT 10